(19) Europäisches Patentamt
European Patent Office
Office européen des brevets

(11) Publication number: **0 024 161**
**A2**

# (12) EUROPEAN PATENT APPLICATION

(21) Application number: 80302685.5

(22) Date of filing: 05.08.80

(51) Int. Cl.$^3$: **A 61 K 7/15**
A 61 K 7/48

(30) Priority: 07.08.79 US 64406

(43) Date of publication of application: 25.02.81 Bulletin 81/8

(84) Designated Contracting States: DE FR GB IT

(71) Applicant: UNION CARBIDE CORPORATION
270, Park Avenue
New York, N.Y. 10017(US)

(72) Inventor: Turney, Mary Elizabeth
151 South State Road
Briarcliff Manor New York 10510(US)

(72) Inventor: McGriff, Sammie Lee
1755 Mountain Avenue
Scotch Plains New Jersey 07076(US)

(74) Representative: McCall, John Douglas et al,
W.P. THOMPSON & CO. Coopers Building Church Street
Liverpool L1 3AB(GB)

## (54) Chemical composition for use in treatment of pseudofolliculitis; method of treatment of pseudofolliculitis and method of shaving to reduce severity of pseudofolliculitis.

(57) There are disclosed chemical compositions for use in treating the dermatological condition known as pseudofolliculitis. The chemical compositions comprise aqueous compositions containing cationic polymers, e.g. cationic quaternary ammonium polymers, said cationic polymers preferably being admixed with various additional agents. There is also disclosed a method of treating the condition known as pseudofolliculitis a method of shaving to reduce the severity of pseudofolliculitis.

EP 0 024 161 A2

Croydon Printing Company Ltd.

## DESCRIPTION

## "CHEMICAL COMPOSITION FOR USE IN TREATMENT OF PSEUDOFOLLICULITIS; METHOD OF TREATMENT OF PSEUDOFOLLICULITIS AND METHOD OF SHAVING TO REDUCE SEVERITY OF PSEUDOFOLLICULITIS"

The present invention relates to novel chemical compositions for use in the treatment of the dermatological condition known as pseudofolliculitis, to methods of treating skin having said same dermatological condition, and to methods of shaving to reduce the severity of pseudofolliculitis.

The present invention generally relates to improved chemical compositions for the treatment and/or prevention of the dermatological condition known as pseudofolliculitis. The chemical compositions of the present invention may be in the form of salves, ointments, lotions or creams and in their preferred form are in the form of cosmetic shaving compositions. These shaving compositions include pre-shaves, shaving creams, shaving gels, after-shaves and the like.

The chemical compositions of the invention contain a cationic polymer and in preferred embodiment may contain a cationic quaternary ammonium polymer, wherein said compositions are advantageously employed by persons having the condition known as pseudofolliculitis, although persons without pseudofolliculitis may also employ these compositions.

Although various forms of pseudofolliculitis are known, the most commonly encountered form is pseudofolliculitis barbae (of the beard). Pseudofolliculitis barbae is defined in the Merck Manual of Diagnosis and Therapy, 13th Edition, 1977, published by Merck Sharp and Dohme Research Laboratories, at 1591, as follows:

"Pseudofolliculitis of the beard (ingrown hairs) is seen almost exclusively in Negro men. The stiff hair tips penetrate into the skin before leaving the follicle, or else leave the follicle, curve, and then reenter nearby skin, provoking small pustules that are more a foreign-body reaction than an infection. The only consistently effective treatment is to have the patient grow a beard. A thioglycolate depilatory may be used every 2 or 3 days but is often irritating. Application of tretinoin (vitamin A acid; retinoic acid) 0.05% liquid or cream daily or every other day may be effective in mild or moderate cases; it is initially irritating and should be used every other day at first, then daily." (Emphasis added)

Although pseudofolliculitis barbae (of the beard) is the most commonly encountered form of pseudofolliculitis other forms are frequently encountered. These other forms include, but are not limited, to pseudofolliculitis pubis (of the pubic area) and pseudofolliculitis capitis (of the scalp).

From the above it can be seen that pseudofolliculitis barbae is a dermatological condition predominantly seen in Negro men, although not limited to Negro men, who shave their facial skin areas to remove facial hair therefrom. This dermatological condition is generally believed to be caused by the end of the facial or other hair curling back into the skin, resulting in a severe inflammatory reaction manifesting itself by "bumps" on the skin from the formation of papules, i.e. elevations of the skin, and pustules, i.e. elevations of the skin which contain pus. The resulting dermatological condition not only results in a very unpleasant appearance of the facial or other skin but also gives a skin condition that causes severe itching and discomfort to the person afflicted with the condition.

The severe dermatological condition resulting from pseudofolliculitis barbae has been documented in a number of publications among these being, "Pseudofolliculitis of the Beard", Strauss, J.S., Kligman, A.M., A.M.A. Archives of Dermatology, Nov. 1956, Vol. 74, pp. 533-542; "Pseudofolliculitis Barbae, 2, Treatment", Brauner, G.J. Flandermeyer, K.L., Int. J. Dermatol, 1977, 16/6 pp. 520-525; "Facial Depilatories on Black Skin", Guardia, M. de la, Cosmetics and Toiletries, Vol. 91, pp. 37, 38 (1976); and "Pseudofolliculitis Barbae in the Military, A Medical, Administrative and Social Problem", Alexander, AM, Delph, W. I., J. Nat. Med. Ass., 1974, 6/6, pp. 459-464.

Prior to the present invention, to the best of our knowledge, the only consistently effective means for treating or preventing pseudofolliculitis barbae was to refrain from shaving the skin and thus permit the growth of a beard on the skin. As aforementioned, some other treatments have been suggested and these include the use of various depilatories and creams. Although these various depilatories and creams have found limited success, the use of these various depilatories and creams have been found to result in skin irritation which further aggravates an already severe dermatological condition.

Alternatively, prior to the present invention, a person afflicted with pseudofolliculitis barbae could physically remove each hair by pulling them out. This method is not only time consuming, but can also be painful.

Thus, in view of the problems associated with the previously known methods of treating pseudofolliculitis barbae, this condition, to the best of our knowledge, has most commonly been "cured" by promptly having the patient cease shaving and thus grow unwanted and often embarrassing hair.

The present invention, unlike the methods of the prior art, treats the condition of pseudofolliculitis by providing chemical compositions, in particular shaving compositions although not limited thereto, which are beneficial in the treatment of pseudofolliculitis. These chemical compositions contain cationic polymers, including in particular the quaternary ammonium polymers, which have been found to relieve the problems associated with pseudofolliculitis.

The present invention relates to chemical compositions and methods of treatment for the dermatological condition known as pseudofolliculitis, wherein the chemical compositions comprise a cationic polymer, preferably a cationic quaternary ammonium polymer, and the methods of treatment comprise the use of said chemical compositions for the treatment of pseudofolliculitis. The present invention also relates to a novel shaving composition which can be used for the treatment of pseudofolliculitis or alternatively may be used for shaving by persons without pseudofolliculitis.

In accordance with the present invention various chemical compositions may be prepared for the treatment and/or prevention of pseudofolliculitis. These chemical compositions include those compositions which are generally called shaving compositions, although chemical compositions other than those commonly referred to as shaving compositions may be prepared in accordance with the present invention.

The chemical compositions of the present invention preferably contain the quaternary ammonium polymers as the cationic polymer, in compositions containing various amphoteric surfactants and cationic and anionic surfactants.

Typical of the various amphoteric surfactants useable herein is the Miranol type surfactants, although other amphoteric surfactants are suitable for use in the present invention. These are substituted imidazolines having as substituents a long chain (i.e. $C_{10}$-$C_{18}$) alkyl radical and radicals containing hydrophilic groups. These surfactants are described in a technical and product development data report, "The Miranol Amphoteric Surface Active Agents", 5th Edition, 1967, published by Miranol Chemical Company Chemical Company Inc.

The preferred amphoteric surfactant of the Miranol type is Miranol C2MSF wherein the long chain alkyl radical is coconut alkyl and the hydrophilic groups are carboxyls.

This amphoteric surfactant has the structure

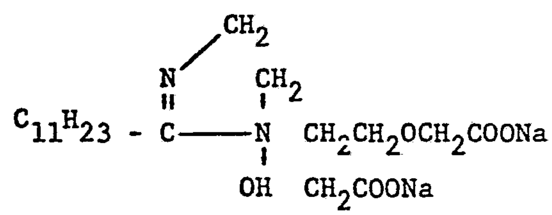

Typical of the anionic surfactants suitable for use in the present invention, but not limited thereto, are triethanolamine lauryl sulfate, ammonium lauryl sulfate, sodium lauryl sulfate and ethoxysulfate.

In addition, the chemical compositions of the invention may contain, in addition to the cationic quaternary ammonium polymers, various other additional agents such as, but not limited thereto, sequestering agents, surfactants, emollients, opacifiers, thickeners, dyes, perfumes, preservatives, conditioners and the like.

In accordance with a preferred embodiment of the present invention a suitable cationic quaternary ammonium polymer, i.e. cationic polymer, is used to prepare a chemical composition for relieving the condition of pseudofolliculitis and in particular as shown in the following examples, a shaving composition is prepared for the use in the treatment of pseudofolliculitis barbae.

Typical of the quaternary nitrogen containing cellulose ethers (hereinafter referred to as QNCC(ethers) which can be used in the present invention are those described in U.S. Patent No. 3,472,840 granted to Stone et al. on October 14, 1969.

These preferred cationic quaternary nitrogen containing cellulose ethers are characterized in U.S. Patent No. 3,472,840, incorporated herein by reference, as those which are embodied within the structural formula:

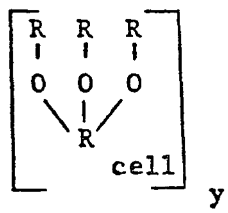

where $R_{cell}$ is the residue of an anhydroglucose unit ($C_6H_{10}O_5$), the R's may be the same or different and each R individually represents a substituent group of the formula given hereinbelow, and y represents the degree of polymerization and is an integer having a value of from about 50 to about 20,000, or more, and preferably from about 200 to about 5,000.

In the above structural formula each R individually represents a substituent group of the general formula:

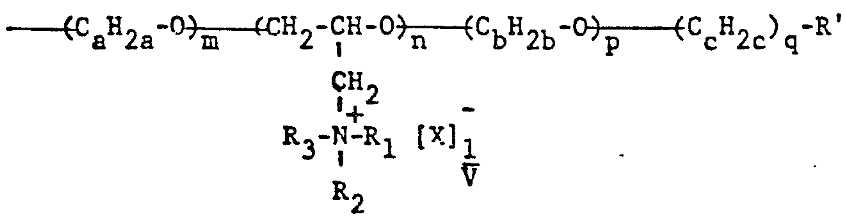

wherein:

a is an integer having a value of from 2 to 3;

b is an integer having a value of from 2 to 3;

c is an integer having a value of from 1 to 3;

m is an integer having a value of from zero to 10;

n is an integer having a value of from zero to 3;

p is an integer having a value of from zero to 10;

q is an integer having a value of from zero to 1;

R' is a member selected from the group consisting of

$-H$, $-\overset{O}{\overset{\|}{C}}-OH$, $-\overset{O}{\overset{\|}{C}}-O-Na$, $-\overset{O}{\overset{\|}{C}}-O-K$ and $-\overset{O}{\overset{\|}{C}}-O-NH_4$ with the proviso that when q is zero then R' is -H;

$R_1$, $R_2$ and $R_3$, taken individually, represent a member selected from the group consisting of alkyl, aryl, aralkyl, alkaryl, cycloalkyl, alkoxyalkyl and alkoxyaryl radicals where each of $R_1$, $R_2$ and $R_3$ can contain up to 10 carbon atoms, with the proviso that when said member is an alkoxyalkyl radical there are at least 2 carbon atoms separating the oxygen atom from the nitrogen atom, and with the further proviso that the total number of carbon atoms in radicals represented by $R_1$, $R_2$ and $R_3$ is from 3 to 12;

$R_1$, $R_2$ and $R_3$, taken together, represent along with the nitrogen atom to which they are attached a member selected from the group consisting of pyridine, α-methylpyridine, 3,5-dimethylpyridine, 2,4,6-trimethylpyridine, N-methyl piperidine, N-ethyl piperidine, N-methyl morpholine and N-ethyl morpholine;

X is an anion such as chloride, bromide, iodide, sulfate, methylsulfate, sulfonate, nitrate, phosphate,

acetate, etc., and V is an integer which is equal to the valence of X;

The average value of n per anhydroglucose unit is from about 0.01 to about 1 and preferably from about 0.1 to about 0.5;

and the average value of m + n + p + q per anhydroglucose unit is from about 0.01 to about 4, more preferably from about 0.1 to about 2.5, and most preferably from about 0.8 to about 2.

Illustrative of the numerous possible pendant groups on the anhydroglucose chain in accordance with the above generic description are the following:

$-CH_3$,

$-CH_2-CH_3$,

$-CH_2-CH_2-OH$,

$-CH_2-CH(CH_3)-OH$,

$-CH_2-CH_2-O-CH_2-CH_2-OH$,

$-CH_2-C(=O)-OH$

$-CH_2-C(=O)-O-Na$,

$-CH_2-CH_2-O-CH_2-CH_2-O-CH_2-CH_2-OH$,

$-CH_2-CH_2-O-CH_2-C(=O)-O-K$,

$-CH_2-CH(CH_2-N^+(CH_3)_3)-O-CH_2-CH_2-OH$ $Cl^-$

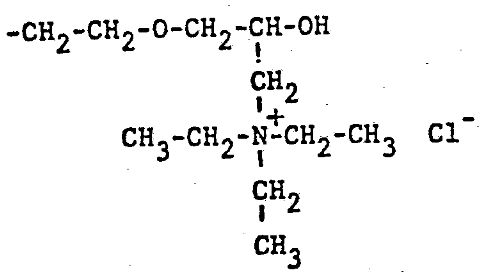

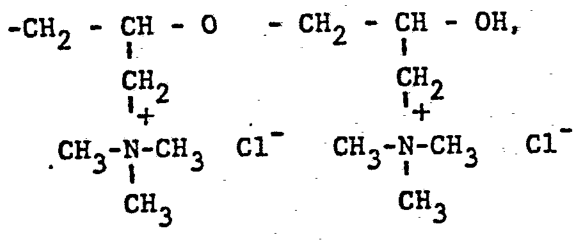

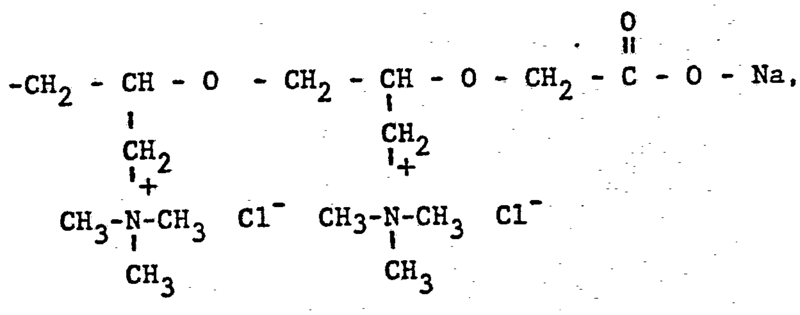

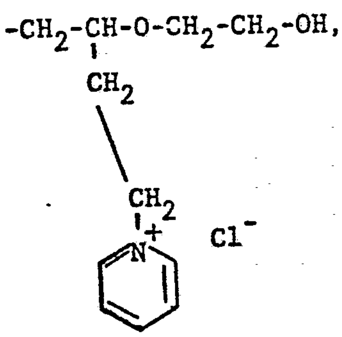

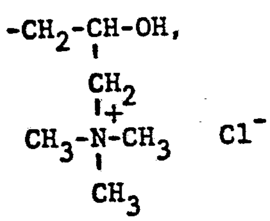

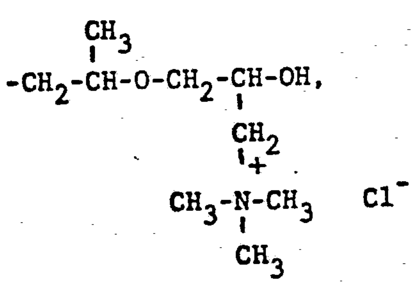

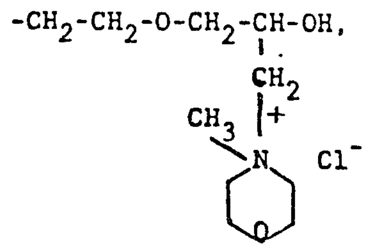

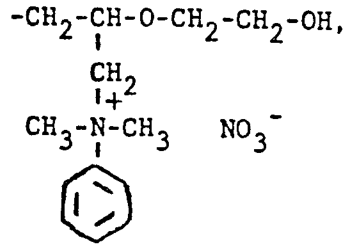

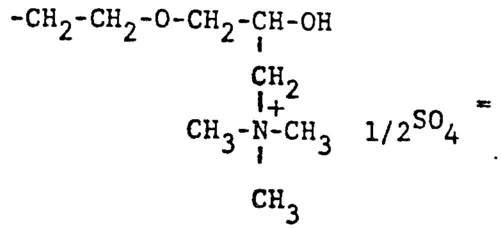

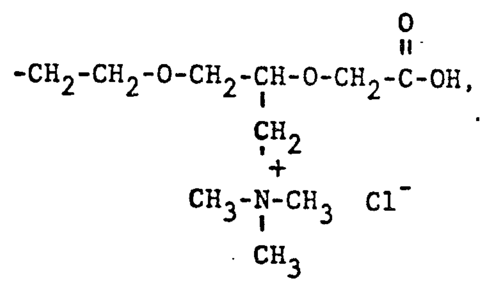

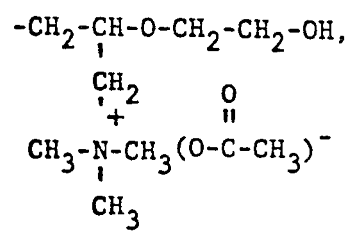

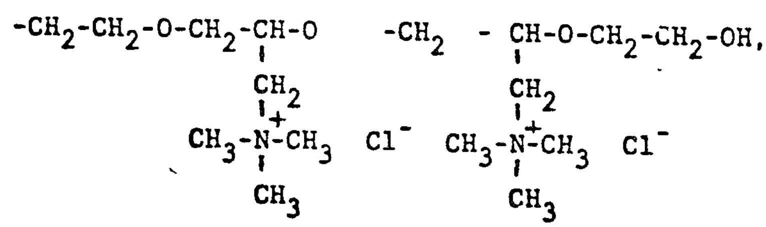

and so forth.

A particularly efficacious quaternary nitrogen-substitute cellulose derivative for the purpose of this invention is available from Union Carbide Corporation under the code designation UCARE "Polymer JR". This polymer has a molecular weight within the range of from 100,000 to 3,000,000. Polymer JR is a cationic cellulose ether having the structure

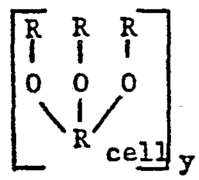

wherein $R_{cell}$ is a residue of an anhydroglucose unit, wherein Y is an integer having values from 50 to 20,000 and wherein each R individually represents a substituent of the general formula:

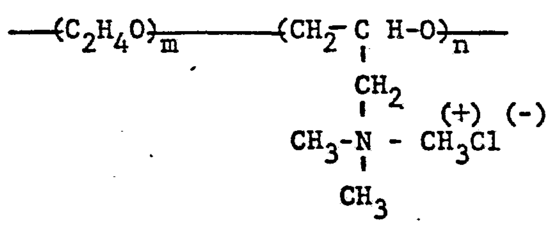

wherein the m is an integer having values from 0 to 10, n is an integer having values from 0 to 10. The average values per anhydroglucose unit are: n is from 0.35 to 0.45 and the sum of m plus p is from 1 to 2.

Among the preferred cationic QNCC ethers for use in the present invention are those having viscosities of 100 to 30,000 centipoises (cps.) at 25°C in 2% by weight aqueous solutions, when measured by ASTM D-2364-65 (Model LVF Brookfield, 30 rpm Spindle No. 2).

Cationic QNCC ethers which are particularly well suited in the present invention are those sold by Union Carbide Corporation under the trade designation UCARE JR-125, UCARE JR-400, and UCARE JR-30M, signifying a polymer of the type described having viscosities of 100 to 125 cps., 400 cps. and 30,000 cps. respectively. UCARE is the registered trademark of Union Carbide Corporation, New York, New York.

In addition to the above-described cationic polymers, other cationic polymers, which possess similar properties for the alleviation of the condition known as pseudofolliculitis, may be employed in the practice of this invention.

It is believed that in addition to the aforementioned quaternary nitrogen containing cellulose ethers that cationic polymers of the amine type can be employed in a manner similar to the aforementioned cationic quaternary nitrogen containing cellulose ether polymers. For example, aliphatic, polyamines, quaternary ammonium polymers of aliphatic polyethers, water-soluble linear polyamines, water soluble polyamines of the reaction product of an aliphatic polyamine in an epoxy such as epichlorhydrin, and the like, are believed to be suitable in the present invention.

Specific illustrations of polymers that are also believed suitable for the present invention, i.e. the treatment of the dermatological condition known as pseudofolliculitis, similar to the treatment provided by the aforementioned quaternary nitrogen containing ether polymers, are, by way of illustration only, as follows:

GAFQUAT-755 (GAFQUATS is the Registered Trade Mark of GAF Corporation, 140 West 51st St., New York, N.Y. 10020) and GAFQUAT-734, a low molecular weight polymer, which are quaternium-23 compounds which are copolymers of vinylpyrrolidone and dimethyl amino ethyl methacrylate quaternized with diethyl sulphate. The GAFQUATS have molecular weights in the range of from about 100,000 to about 1,000,000 with GAFQUAT-734 having a molecular weight of about 100,000 and is available as a 50% by weight solution in ethanol, and GAFQUAT-755 which has a molecular weight of about 1,000,000 and is available as a 20% by weight aqueous solution;

MERQUAT (MERQUAT is the Registered Trade Mark of Merck and Company, Inc., Rahway, New Jersey), which is a polymer sold under the designations MERQUAT-100 and MERQUAT-550, both of which are polymers of N, N-dimethyl-3,5-methylene piperdinium chloride. In particular, MERQUAT-100 is a homopolymer and MERQUAT-550 is a copolymer with acrylamide; and

JAGUAR C-13 (JAGUAR is a Registered Trade Mark of Celanese Polymer Specialties Company, Louisville, Kentucky 40202), which is a low viscosity cationic quar gum derivative.

The compositions of the present invention are generally formulated to contain from about 0.2 to about 5% by weight of the cationic polymer although over 5% may be used.

The invention will be more fully understood from the examples which follow and, in particular the formulations of the cosmetic compositions in examples 1-3. These examples are given by way of illustration and are not to be considered as limiting. In addition, the following examples particularly refer to pseudofolliculitis barbae although the use of these various compositions as well as others containing cationic polymers is equally applicable to the treatment of other forms of pseudofolliculitis.

Example 1

In accordance with the present invention, a chemical composition is prepared having the following formulation:

| | Percent by Weight |
|---|---|
| Miranol C2MSF | 7.5 |
| Triethanolamine lauryl alcohol | 7.5 |
| Lauric diethanolamide | 2.0 |
| Polymer JR-400 | 1.0 |
| Water, preservatives | q.s. |

Example 2

In accordance with the present invention, a chemical composition is prepared having the following formulation:

| | Percent by Weight |
|---|---|
| Triethanolamine lauryl sulfate | 20 |
| Polymer JR-30M | 1.5 |
| Polymer JR-400 | 0.1 |
| Lauric diethanolamide | 2.0 |
| Water, preservatives | q.s. |

Example 3

In accordance with the present invention a shaving composition in the form of a gel is prepared having the following formulation:

| | Percent by Weight |
|---|---|
| Triethanolamine lauryl sulfate | 10.0 |
| Disodium sulphosuccinate of lauryl polyglycol ether | 5.0 |
| Tween 20 (Polyoxyethylene 20 sorbitan monolaurate) | 2.0 |
| Polymer JR-30M | 1.75 |
| Polymer JR-125 | 0.25 |
| Methyl Paraben (methyl p-hydroxybenzoate) | 0.2 |
| Propyl Paraben (propyl p-hydroxybenzoate) | 0.05 |
| Germall (imidiazolidinyl urea) | 0.75 |
| Water, dye, fragrance | q.s. |

Example 4

A moisturizing cream is prepared by preparing an oil phase and a water phase as follows:

| Oil Phase | Percent by Weight |
|---|---|
| Mineral Oil | 2.5 |
| Isocetyl Stearate | 1.5 |
| Isopropyl Myristate | 1.0 |
| Stearic Acid | 2.9 |
| Ethylene Glycol Monostearate | 1.0 |
| Olive Oil | 0.1 |
| Lanolin | 0.5 |
| Propyl Paraben | 0.05 |
| Squalene | 0.1 |

| Water Phase | Percent by Weight |
|---|---|
| Propylene Glycol | 4.0 |
| Triethanolamine | 0.95 |
| Methyl Paraben | 0.1 |
| Polymer JR-400 | 0.2 |
| Water, dye, fragrance | q.s.. |

The moisturizing cream is then prepared by the following procedure:

1. Heat the oil phase to 70°C.
2. In a separate container, add the Polymer JR-400 to the available water. When the polymer is hydrated, add the remaining water soluble ingredients and heat to 70°C.
3. Add the water phase to the oil phase while stirring vigorously.
4. When the temperature reaches about 40°C, the perfume and coloring are added.
5. Continue the stirring until the temperature of the lotion is 35°C or less.

Example 5

A shaving composition, comprising an after shave, is prepared as follows:

| | Percent by Weight |
|---|---|
| Polymer JR-125 | 0.5 |
| Ethanol, SD40 | 55.0 |
| Methyl Paraben | 0.1 |
| Water, Fragrance | q.s. |

The after shave is prepared by dissolving the methyl paraben in the water. To this mixture is added the Polymer JR-125. When the mixture is complete the alcohol and fragrance are added.

Example 6

A chemical composition comprising a cream is prepared by preparing an oil phase and a water phase as follows:

Oil Phase

| | Percent by Weight |
|---|---|
| Mineral Oil | 26.0 |
| Lanolin | 3.0 |
| Cetyl Alcohol | 0.5 |
| Petrolatum | 7.0 |
| Beeswax | 2.0 |
| Polawax | 1.0 |
| Arlacel 165 | 3.0 |
| Propyl Paraben | 0.05 |

Water Phase

| | Percent by Weight |
|---|---|
| Myrj-52 | 3.0 |
| Propylene Glycol | 4.0 |
| Borax | 0.5 |
| Polymer JR-400 | 0.5 |
| Germall | 0.5 |
| Methyl Paraben | 0.2 |
| Water, dye, perfume | q.s. |

The chemical composition is then prepared by the following procedure:

1. Heat the oil phase to 70°C.
2. In a separate container, add the Polymer JR-400 to the available water. When the polymer is hydrated, add the remaining water soluble ingredients and heat to 70°C.
3. Add the water phase to the oil phase while stirring vigorously.
4. When the temperature reaches about 40°C, the perfume and coloring are added.
5. Continue the stirring until the temperature of the composition is 35°C or less.

## Example 7

A chemical composition in the form of a lotion is prepared by preparing an oil phase and a water phase as follows:

### Oil Phase

| | Percent by Weight |
|---|---|
| Di(2-Ethylhexyl) Adipate | 4.80 |
| Stearic Acid | 2.90 |
| "Nimcolan T" (Malmstrom Chemical Corp.) | 0.50 |
| Cetyl Alcohol | 0.40 |
| Glycerol Monostearate | 1.00 |
| Propyl p-Hydroxybenzoate | 0.05 |

### Water Phase

| | Percent by Weight |
|---|---|
| Triethanolamine, 99% | 0.95 |
| Propylene Glycol | 4.80 |
| Polymer JR-400 | 0.20 |
| Methyl p-Hydroxybenzoate | 0.10 |
| Water | 83.90 |
| Perfume | 0.40 |

The chemical composition is then prepared as follows:

1. Heat the oil phase to 70°C.
2. In a separate container, add the Polymer JR-400 to 10 percent water and stir until hydrated.
3. In a third container, heat the remaining water, triethanolamine, propylene glycol, and methyl p-hydroxybenzoate to 70°C.

4. Add the water phase and then the Polymer JR-400 solution to the oil phase while stirring vigorously.
5. Continue the stirring at a moderate rate until the temperature reaches 40°C., when the perfume is added.
6. Stirring is ceased when the temperature reaches 35°C. or lower. Viscosity-1,800 to 2,200 cps. at 25°C. (Brookfield RVT, 50 rpm., Spindle No. 5)

Example 8

A shaving composition as in Example 3 is prepared, except that instead of using the cationic polymers such as JR-30M and JR-125 the non-cationic polymers CELLOSIZE 52000 and CELLOSIZE 4400 were substituted for cationic polymers JR-30M and JR-125, respectively.

CELLOSIZE is the Registered Trademark of Union Carbide Corporation for polymers of hydroxyethylcellulose and CELLOSIZE 52000 and CELLOSIZE 4400 are designations of said polymers with viscosities of 52000 cps. and 4400 cps., respectively.

Example 9

The shaving composition prepared in Example 3 was tested by a human subject having pseudofolliculitis barbae, said subject being designated subject A who applied the composition to his face and then shaved with a blade razor the skin which was afflicted with pseudofolliculitis barbae.

Subject A reported that the composition of Example 3 reduced irritation associated with pseudofolliculitis barbae.

Example 10

The shaving composition of Example 3 was tested for the treatment of pseudofolliculitis barbae on seven human subjects suffering from pseudofolliculitis barbae. Originally, nine subjects were tested but two subjects did not complete the testing period.

Each subject was initially inspected to determine his initial facial condition. Each subject was then instructed to wet his face, apply a small quantity of the composition of Example 3 and shave with a blade-type razor. Each subject was instructed to shave as often as comfortable.

The subjects all showed improvement in their skin condition as measured by a decrease in the number of bumps on the face and the amount of irritations experienced during shaving. The responses of each subject, designated by the letters B, C, D, E, F, G and H, were as follows:

Subject B had an initially moderate case of pseudofolliculitis on his cheeks and a more severe case under his chin. After one week of shaving with the composition of Example 3 Subject B reported that his condition was improving and fewer bumps were present. After three months Subject B reported that he experienced none of the itching that had previously occurred in summer months. Subject B described the composition superior to those previously used by him.

Subject C initially had a severe case of pseudofolliculitis barbae. After using the composition of Example 3 for three days Subject C reported that some of the bumps were disappearing. After one week Subject C reported a noticeable improvement in his condition and after one month he reported continued improvement and the absence of itching that had previously occurred in summer months. After three months Subject C reported that the absence of itching continued and that the composition was superior to those achieved previously under the advice of a dermatologist.

Subject D initially had a minor case of pseudofolliculitis barbae. After one week of shaving with the composition of Example 3 Subject D reported that irritation of his facial regions had decreased and that the bumps were disappearing. After one month Subject C reported his condition continued to improve.

Subject E initially had a severe case of pseudofolliculitis barbae. After five days Subject E reported his skin was sore and the razor was hard to rinse. After seventeen days Subject E reported a noticeable improvement in his skin but that he still cut himself (possibly indicating some bumps still present). After 3 months Subject E reported that his dermatological condition was much improved, he was no longer cutting himself and he preferred to use the composition of Example 3 by placing it on his face for one hour or so prior to shaving.

Subject F initially had a minor case of pseudo-follculitis barbae. After one week Subject F reported that his face had fewer bumps, he experienced no nicking while shaving and he felt his skin was smooth. After one month Subject F reported that he felt the composition of Example 3 was superior to other shaving compositions he had tried and after three months Subject F reported that the bumps on this facial regions were gone.

Subject G initially had a moderate case of pseudofolliculitis barbae. After use of the compositions of Example 3 Subject G reported that the composition was superior to other shaving compositions he had used and it allowed him to shave at least every other day with a sharp razor blade rather than a dull razor blade.

Subject H initially had a minor case of pseudofolliculitis barbae. After two weeks Subject H reported a noticeable improvement and after five weeks further improvement was reported. After two months Subject H reported further improvement of his skin condition.

Example 11

The shaving compositions of Example 3 and Example 8 were tested by six human subjects having pseudofolliculitis barbae by use of blind half-face tests, wherein the compositions of Example 3 and Example 8 were each tested on one half of the subject's face.

After the tests each subject was asked to choose which composition gave better results, e.g. was less irritating to use in shaving and reduced the number

of bumps on the subject's face. All six subjects selected the composition of Example 3 as superior. In fact, several subjects reported that the composition of Example 8 produced irritation of their skin and thus irritated their pseudofolliculitis barbae.

Example 12

The shaving compositions of Example 3 and Example 8 were tested again as in Example 7 but in a blind study using ten human subjects having cases of pseudofolliculitis barbae. Three subjects shaved only once and discontinued the study. The remaining seven subjects continued shaving for two weeks, the duration of the study.

At the end of the study it was observed that the composition of Example 3 provided a greater benefit, within the two week duration of the study, to the more moderate cases of pseudofolliculitis barbae.

Example 13

The shaving composition of Example 3 was tested on a group of seventeen human subjects who were instructed to apply the composition and shave with a blade-type razor. Of these seventeen subjects only eight subjects completed the eight week period of the study.

The subjects were initially evaluated as to degree of pseudofolliculitis of each subject. Each subject was then "graded" as set forth in Table I for the eight subjects completing the study.

TABLE I

| Subject | Initial Evaluation[a] |
|---|---|
| 1 | III |
| 2 | II |
| 3 | II to III |
| 4 | II to III |
| 5 | II |
| 6 | I |
| 7 | II |
| 8 | I |

a The grading used in the initial evaluation is as follows:

| Grade | Interpretation | Shaving Difficulty |
|---|---|---|
| 0 | No evidence of pseudo-folliculitis barbae | None |
| I | Ingrown hairs and less than 20 papules of 2mm or greater diameter | Minor |
| II | Ingrown hairs plus 20 or more papules of 2mm or greater diameter | Moderate |
| III | Grade II plus multiple pustules | Severe |
| IV | Grade 2 or 3 plus abscess formation | Very Severe |

The grading was based on a "shaving wedge", i.e. an area of the face, corresponding to about one half of the subject's face.

The composition of Example 3 was found to be effective in reducing the irritation and inflammation associated with pseudofolliculitis barbae in each of the eight subjects completing the eight week study.

CLAIMS

1. A composition for use in a method of treatment of pseudofolliculitis, said composition characterised by comprising an aqueous mixture of a cationic polymer effective in the treatment of pseudofolliculitis.

2. A composition as claimed in claim 1 or 2, characterised in that said composition contains an amphoteric surface active agent.

3. A composition as claimed in claim 1 or 2, characterised in that said composition contains a perfume and a dye.

4. A composition as claimed in any one of claims 1 to 3, characterised in that said composition contains a preservative.

5. A composition as claimed in any one of the preceding claims, wherein the cationic polymer is a cationic quaternary nitrogen containing cellulose ether.

6. A composition as claimed in claim 5, characterised in that the cationic quaternary nitrogen containing cellulose ether is a polymeric quaternary cellulose ether of the structural formula:

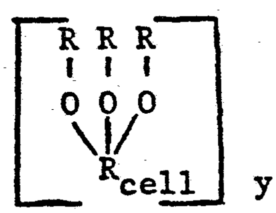

wherein $R_{cell}$ is the residue of an anhydroglucose unit, y is an integer having a value of from about 50 to about 20,000, and each R individually represents a substituent group of the general formula:

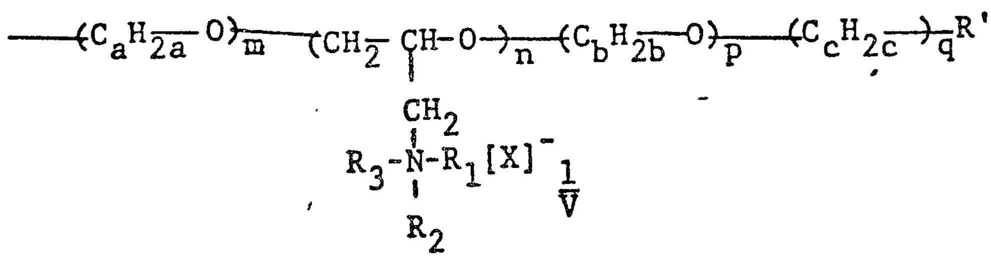

wherein:

a is an integer having a value of from 2 to 3;

b is an integer having a value of from 2 to 3;

c is an integer having a value of from 1 to 3;

m is an integer having a value of from zero to 10;

n is an integer having a value of from zero to 3;

p is an integer having a value of from zero to 10;

q is an integer having a value of from zero to 1;

R' is a member selected from the group consisting of

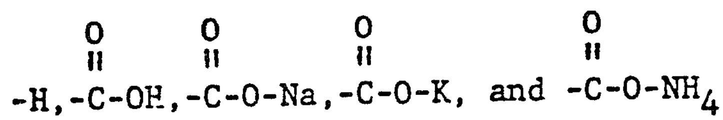

with the proviso that when q is zero then R' is -H; $R_1$, $R_2$ and $R_3$, taken individually, represent a member selected from the group consisting of alkyl, aryl, aralkyl, alkaryl, cycloalkyl, alkoxyalkyl and alkoxyaryl radicals where each of $R_1$, $R_2$ and $R_3$ can contain up to 10 carbon atoms, with the proviso that when said member is an alkoxyalkyl radical there are at least 2 carbon atoms separating the oxygen atom from the nitrogen atom, and with the further proviso that the total number of carbon atoms in radicals represented by $R_1$, $R_2$ and $R_3$ is from 3 to 12 with the further proviso that when $R_1$, $R_2$ and $R_3$ are taken together the nitrogen atom to which $R_1$, $R_2$ and $R_3$ are attached can be a component of a heterocyclic ring selected from the group consisting of pyridine, α-methylpyridine, 2,5-dimethylpyridine, 2,4,6-trimethylpyridine, N-methyl piperidine, N-ethyl piperidine, N-methyl morpholine and N-ethyl morpholine; X is an anion; V is an integer which is equal to the valence of X; the average value of n per anhydroglucose unit of said cellulose ether is from about 0.01 to about 1; and the average value of m+p+q per anhydroglucose unit of said cellulose ether is from about 0.01 to about 4.

7. A composition as claimed in any one of the preceding claims, characterised in that said cationic polymer is present in an amount of about 0.2% to 5% by weight.

8. A composition as claimed in any one of the preceding claims, characterised in that said aqueous composition comprises about 5.0% by weight of the disodium sulphosuccinate of lauryl polyglycol ether and from about 0.2% to 5.0% by weight of a cationic quaternary nitrogen containing cellulose ether.

9. A method of treatment of pseudofolliculitis, characterised by the step of applying to an effected area a composition as claimed in any one of the preceding claims, and repeating said step until a desired decrease in severity of pseudofolliculitis is achieved.

10. A method of shaving to reduce the serverity of pseudofolliculitis comprising applying a shaving composition to the skin and shaving off unwanted hair with a blade type razor, characterised in that said shaving composition is a composition as claimed in any one of claims 1 to 9.